# EUROPEAN PATENT SPECIFICATION

(11) **EP 3 851 026 B1**
(45) Date of publication and mention of the grant of the patent: **09.10.2024**
(21) Application number: 19858982.2
(22) Date of filing: 05.08.2019
(51) Int. Cl.: A61B 1/06, A61B 1/00, A61B 1/045, G02B 23/24, G06T 7/00

(54) **ENDOSCOPE DEVICE, ENDOSCOPE PROCESSOR, AND ENDOSCOPE DEVICE OPERATION METHOD**
ENDOSKOPVORRICHTUNG, ENDOSKOPPROZESSOR UND ENDOSKOPVORRICHTUNGSBETRIEBSVERFAHREN
DISPOSITIF D'ENDOSCOPE, PROCESSEUR D'ENDOSCOPE ET PROCÉDÉ DE FONCTIONNEMENT DE DISPOSITIF D'ENDOSCOPE

(30) Priority: 12.09.2018 JP 2018170762
(43) Date of publication of application: 21.07.2021
(73) Proprietor: FUJIFILM Corporation, Tokyo 106-8620 (JP)
(72) Inventor: OOSAKE, Masaaki, Ashigarakami-gun, Kanagawa 258-8538 (JP)
(74) Representative: Dehns Germany Partnerschaft mbB
(86) International application number: PCT/JP2019/030732
(87) International publication number: WO 2020/054255

(56) References cited:
- WO-A1-2017/199509
- WO-A1-2017/199509
- US-A1- 2019 069 769

## Description

### BACKGROUND OF THE INVENTION

### 1. Field of the Invention

The present invention relates to an endoscope apparatus, an endoscope processor, and a method for operating the endoscope apparatus, and more specifically to a technique for reducing the burden of a user's operation of an endoscope.

### 2. Description of the Related Art

A typical endoscope apparatus irradiates an observation target with illumination light emitted from a distal end of an insertion section of an endoscope and captures an image of the observation target by using an imaging element to acquire image information. It is known that the illumination light can be implemented as special light, as well as white light (normal light), having a different spectrum from white light (JP2012-000160A and JP2014-166590A).

The endoscope apparatus described in JP2012-000160A has a probe portion at the distal end of the insertion section of the endoscope such that the probe portion is pressed against a surface of a living body to detect a feature value of the surface of the living body, and automatically switches an observation mode (illumination light) between a normal-light observation mode using white light and a special-light observation mode using special light in accordance with the detected feature value.

The endoscope apparatus described in JP2014-166590A has a first illumination mode in which the amount of narrow-band light is increased compared to the amount of broadband light, a second illumination mode in which the amount of narrow-band light is substantially equal to the amount of broadband light, and a third illumination mode in which the amount of narrow-band light is decreased compared to the amount of broadband light, determines the type of an observation site, and automatically switches the illumination mode in accordance with the determined type of the observation site, thereby reducing the load on the operator.

In recent years, it has been known to support an examination by performing recognition such as detecting a lesion included in an image through image analysis or classifying lesions by type and by performing notification.

In image analysis for recognition, accurate automatic recognition is enabled by machine learning of images such as deep learning (for example, A. Krizhevsky, I. Sutskever, and G. Hinton, ImageNet classification with deep convolutional neural networks, in NIPS, 2012).

### SUMMARY OF THE INVENTION

In the invention described in JP2012-000160A, the probe portion at the distal end of the insertion section of the endoscope is pressed against a surface of a living body to dent the surface of the living body, and when the size of the dented region of the surface of the living body exceeds a threshold value, the observation mode is automatically switched to the special-light observation mode. The operator needs to press the probe portion against the surface of the living body (perform a palpation).

In the invention described in JP2014-166590A, in accordance with the type of the observation site (for example, the esophagus, the cardia, or the stomach), automatic switching is performed among the first illumination mode using illumination light suitable for special-light observation of the esophagus, the second illumination mode using illumination light suitable for special-light observation of the cardia, and the third illumination mode using illumination light suitable for special-light observation of the stomach. The automatic switching is performed only for the observation of a plurality of observation sites of different types in a single endoscopic examination.

WO 2017/199509 discloses a living body observation system that switches from a deep blood vessel mode to a white light mode when the size of a bleeding region is less than a second predetermined value during treatment. If the size of the bleeding becomes more than a first predetermined value during the treatment, the system switches from the white light mode to the deep blood vessel mode.

The present invention has been made in view of such circumstances, and an object thereof is to provide an endoscope apparatus, an endoscope processor, and a method for operating the endoscope apparatus in which illumination light (observation mode) is automatically switched in response to detection of a detection target from an image to reduce the burden of an operator's switching operation.

In an aspect of the present invention, there is provided an endoscope apparatus as claimed in claim 1.

According to the aspect of the present invention, in response to continuous detection of the detection target from images captured under the first illumination light in the first observation mode, the first observation mode is automatically switched to the second observation mode in which an image is captured under the second illumination light. Thus, it is possible to capture an image of the detection target under the second illumination light, which is suitable for detailed observation of the detection target, and to reduce the burden of the operator's operation of switching the observation mode.

When the amount of change is within the threshold value, it is considered that the image remains substantially stationary (the operator is gazing at the detection target). Thus, the observation mode is switched to the second observation mode, which is suitable for detailed observation of the detection target.

In an endoscope apparatus according to still another aspect of the present invention, preferably, the specific region is an entire region of an image captured by the imaging unit.

In an endoscope apparatus according to still another aspect of the present invention, preferably, the specific region is a center region of an image captured by the imaging unit.

In an endoscope apparatus according to still another aspect of the present invention, preferably, the specific region is a region corresponding to the detection target, the region being calculated based on detection information from the detector.

In an endoscope apparatus according to still another aspect of the present invention, preferably, the amount of change calculated by the amount-of-change calculation unit is an amount of change in a position of the specific region.

In an endoscope apparatus according to still another aspect of the present invention, preferably, the amount of change calculated by the amount-of-change calculation unit is an amount of change in a size of the specific region.

In an endoscope apparatus according to still another aspect of the present invention, preferably, in response to an elapse of a certain period of time after the observation mode switching unit switches to the second observation mode, the observation mode switching unit switches to the first observation mode. This is because the observation of the detection target in the second observation mode is completed after a certain period of time has elapsed.

In an endoscope apparatus according to still another aspect of the present invention, preferably, after the observation mode switching unit switches to the second observation mode, the observation mode switching unit switches to the first observation mode in response to the continuous detection determination unit determining that the detection target is not continuously detected. This is because there is no detection target to be observed in the second observation mode.

In an endoscope apparatus according to still another aspect of the present invention, preferably, after the observation mode switching unit switches to the second observation mode, the observation mode switching unit switches to the first observation mode in response to the amount-of-change determination unit determining that the amount of change is larger than the threshold value. When the amount of change is larger than the threshold value, it is considered that the image changes and the operator is not gazing at the detection target. Thus, the observation mode is switched to the first observation mode.

In an endoscope apparatus according to still another aspect of the present invention, preferably, after the observation mode switching unit switches to the second observation mode, the observation mode switching unit switches to the first observation mode in response to a still image being captured.

In an endoscope apparatus according to still another aspect of the present invention, preferably, the continuous detection determination unit determines that the detector continuously detects the detection target in response to the detector consecutively detecting the detection target within a certain time range longer than a detection interval of the detector.

In an endoscope apparatus according to still another aspect of the present invention, preferably, the continuous detection determination unit determines that the detector continuously detects the detection target in response to the detector detecting the detection target at a rate greater than or equal to a threshold value within a certain time range longer than a detection interval of the detector.

In an endoscope apparatus according to still another aspect of the present invention, preferably, the first observation mode is a normal-light observation mode in which normal light is used as the first illumination light, and the second observation mode is a special-light observation mode in which special light is used as the second illumination light.

In an endoscope apparatus according to still another aspect of the present invention, preferably, the first observation mode is a first special-light observation mode in which first special light is used as the first illumination light, and the second observation mode is a second special-light observation mode in which second special light different from the first special light is used as the second illumination light.

According to the present invention, in response to continuous detection of a detection target from images captured under first illumination light in a first observation mode, the observation mode is automatically switched to a second observation mode in which an image is captured under second illumination light. Thus, it is possible to capture an image of the detection target under the second illumination light, which is suitable for detailed observation of the detection target, and to reduce the burden of the operator's operation of switching the observation mode.

### BRIEF DESCRIPTION OF THE DRAWINGS

Fig. 1 is a perspective view illustrating the external appearance of an endoscope apparatus 10 according to the present invention;
Fig. 2 is a block diagram illustrating an electric configuration of the endoscope apparatus 10;
Fig. 3 is a schematic diagram illustrating a typical example configuration of a convolutional neural network, which is one of the learning models constituting a detector 15;
Fig. 4 is a block diagram illustrating a main part of a non-claimed first embodiment of an endoscope processor in an endoscope apparatus according to the present invention;
Fig. 5 is a conceptual diagram illustrating automatic switching of an observation mode when a normal-light observation mode using WL is set as a first observation mode and a special-light observation mode using special light for BLI is set as a second observation mode;
Fig. 6 is a block diagram illustrating a main part of a second embodiment of an endoscope processor in an endoscope apparatus according to the present invention;
Fig. 7 is a block diagram illustrating a main part of a third embodiment of an endoscope processor in an endoscope apparatus according to the present invention;
Fig. 8 is a diagram illustrating input images (frames) sequentially captured by an endoscope 11, detection results of a detection target detected from the input images, amounts of change in the center coordinates of the detection target, and amounts of change in the size (area) of the detection target;
Fig. 9 is a conceptual diagram illustrating automatic switching of the observation mode when a first special-light observation mode using special light for BLI is set as the first observation mode and a second special-light observation mode using special light for LCI is set as the second observation mode; and
Fig. 10 is a flowchart illustrating an embodiment of a method for operating an endoscope apparatus according to the present invention.

### DESCRIPTION OF THE PREFERRED EMBODIMENTS

The following describes preferred embodiments of an endoscope apparatus, an endoscope processor, and a method for operating the endoscope apparatus according to the present invention with reference to the accompanying drawings.

### [Overall Configuration of Endoscope Apparatus]

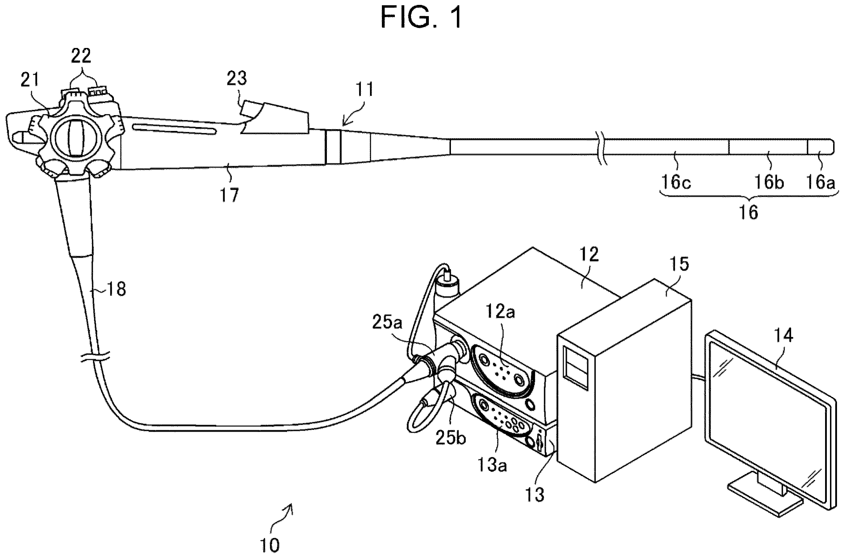

Fig. 1 is a perspective view illustrating the external appearance of an endoscope apparatus 10 according to the present invention.

As illustrated in Fig. 1, the endoscope apparatus 10 is constituted mainly by an endoscope (here, a flexible endoscope) 11 that captures an image of an observation target in a subject, a light source device (light source unit) 12, an endoscope processor 13, a display device 14 such as a liquid crystal monitor, and a detector 15.

The light source device 12 supplies various types of observation light, including white light (first illumination light) for capturing a normal-light image and special light (second illumination light) having a different spectrum from white light, to the endoscope 11.

The endoscope processor 13 has an image processing function for generating image data of a normal-light image, a special-light image, or an observation image to be used for display/recording based on an image signal obtained by the endoscope 11, a function of controlling the light source device 12, a function of causing the display device 14 to display the normal-light image or the observation image and a detection result obtained by the detector 15, and so on.

As described in detail below, the detector 15 is a section that accepts an endoscopic image from the endoscope processor 13 and detects the position of a detection target (such as a lesion, a scar from an operation, or a scar after a treatment) from the endoscopic image, discriminates the type of the lesion, or performs other processing. In this example, the endoscope processor 13 and the light source device 12 are constructed separately and electrically connected to each other. Alternatively, the light source device 12 may be incorporated into the endoscope processor 13. Likewise, the detector 15 may be incorporated into the endoscope processor 13.

The display device 14 displays a normal-light image, a special-light image, or an observation image based on image data to be used for display that is input from the endoscope processor 13, and a recognition result obtained by the detector 15.

The endoscope 11 includes a flexible insertion section 16 to be inserted into a subject, a handheld operation section 17 coupled to a proximal end portion of the insertion section 16 and used to grasp the endoscope 11 and operate the insertion section 16, and a universal cord 18 that connects the handheld operation section 17 to the light source device 12 and the endoscope processor 13.

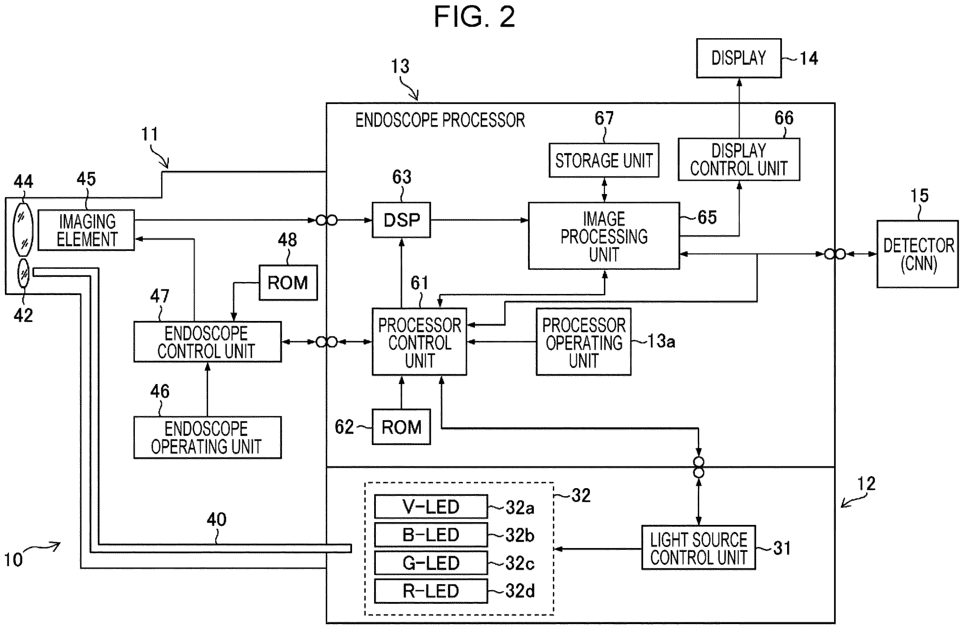

An insertion section distal end 16a at a distal end of the insertion section 16 incorporates an illumination lens 42, an objective lens 44, an imaging element 45, and so on (see Fig. 2). A bendable bending portion 16b is coupled to the rear end of the insertion section distal end 16a. A flexible pipe portion 16c having flexibility is coupled to the rear end of the bending portion 16b.

The handheld operation section 17 is provided with an angle knob 21, an operation button 22, a forceps inlet 23, and so on. The angle knob 21 is rotated to adjust the bending direction and the amount of bending of the bending portion 16b. The operation button 22 is used for various operations such as air supply, water supply, and suction. The forceps inlet 23 communicates with a forceps channel in the insertion section 16. The handheld operation section 17 is also provided with an endoscope operating unit 46 (see Fig. 2) that performs various kinds of setting, and so on.

The universal cord 18 has installed therein an air/water supply channel, a signal cable, a light guide, and so on. The universal cord 18 has disposed in a distal end portion thereof a connector portion 25a to be connected to the light source device 12 and a connector portion 25b to be connected to the endoscope processor 13. Accordingly, observation light is supplied from the light source device 12 to the endoscope 11 via the connector portion 25a, and an image signal obtained by the endoscope 11 is input to the endoscope processor 13 via the connector portion 25b.

The light source device 12 is provided with a light source operating unit 12a such as a power button, a turn-on button for turning on the light source, and a brightness adjustment button, and the endoscope processor 13 is provided with a processor operating unit 13a including a power button and an input unit for accepting input from a pointing device such as a mouse (not illustrated).

### [Electric Configuration of Endoscope Apparatus]

Fig. 2 is a block diagram illustrating an electric configuration of the endoscope apparatus 10.

As illustrated in Fig. 2, the endoscope 11 roughly has a light guide 40, the illumination lens 42, the objective lens 44, the imaging element 45, the endoscope operating unit 46, an endoscope control unit 47, and a ROM (Read Only Memory) 48.

Examples of the light guide 40 include a large-diameter optical fiber and a bundle fiber. The light guide 40 has a light incident end that is inserted into the light source device 12 via the connector portion 25a, and a light emitting end that passes through the insertion section 16 and faces the illumination lens 42 disposed in the insertion section distal end 16a. Illumination light supplied from the light source device 12 to the light guide 40 is applied to the observation target via the illumination lens 42. The illumination light reflected and/or scattered by the observation target is incident on the objective lens 44.

The objective lens 44 forms an image of reflected light or scattered light of the incident illumination light (i.e., an optical image of the observation target) on an imaging surface of the imaging element 45.

The imaging element 45 is a CMOS (complementary metal oxide semiconductor) or CCD (charge coupled device) imaging element and is positioned and fixed relatively to the objective lens 44 at a position on the back side of the objective lens 44. On the imaging surface of the imaging element 45, a plurality of pixels constituted by a plurality of photoelectric conversion elements (photodiodes) that perform photoelectric conversion of an optical image are arranged two-dimensionally. In this example, on the light incident surface side of the plurality of pixels of the imaging element 45, red (R), green (G), and blue (B) color filters are arranged for the respective pixels, thereby forming an R pixel, a G pixel, and a B pixel. The filter arrangement of the RGB color filters is typically, but not limited to, a Bayer arrangement.

The imaging element 45 converts the optical image formed by the objective lens 44 into an electrical image signal and outputs the electrical image signal to the endoscope processor 13.

When the imaging element 45 is a CMOS imaging element, an A/D (Analog/Digital) converter is incorporated, and a digital image signal is output from the imaging element 45 directly to the endoscope processor 13. When the imaging element 45 is a CCD imaging element, an image signal output from the imaging element 45 is converted into a digital image signal by an A/D converter (not illustrated) or the like and is then output to the endoscope processor 13.

The endoscope operating unit 46 has arranged thereon a still-image capturing button (not illustrated) and a mode switching button (not illustrated) for manually switching an observation mode, and a switching signal from the mode switching button is input to the endoscope control unit 47. The mode switching button is an operating unit that switches the type of illumination light (observation mode) each time the mode switching button is pressed, and includes an "AUTO" mode for automatically switching the observation mode, as described below. The mode switching button may be disposed in the processor operating unit 13a of the endoscope processor 13.

The endoscope control unit 47 sequentially executes various programs and data read out from the ROM 48 or the like in accordance with the operation performed using the endoscope operating unit 46, and mainly controls driving of the imaging element 45. For example, in the normal-light observation mode in which white light (normal light) is used as illumination light, the endoscope control unit 47 controls the imaging element 45 to read out signals of the R pixel, the G pixel, and the B pixel of the imaging element 45. In the special-light observation mode in which special light having a different spectrum from white light is used as illumination light, when violet light is emitted from a V-LED 32a or blue light is emitted from a B-LED 32b as observation light to acquire a specific special-light image, the endoscope control unit 47 controls the imaging element 45 to read out signals of only the B pixel of the imaging element 45 having spectral sensitivity in the wavelength range of violet light or blue light or to read out any one color pixel or two color pixels among the three color pixels including the R pixel, the G pixel, and the B pixel.

Further, the endoscope control unit 47 communicates with a processor control unit 61 of the endoscope processor 13 and transmits to the endoscope processor 13 information on the operation performed by the endoscope operating unit 46, identification information for identifying the type of the endoscope 11 stored in the ROM 48, and the like.

The light source device 12 has a light source control unit 31 and a light source unit 32. The light source control unit 31 controls the light source unit 32 and communicates with the processor control unit 61 of the endoscope processor 13 to exchange various kinds of information.

The light source unit 32 has, for example, a plurality of semiconductor light sources. In this embodiment, the light source unit 32 has LEDs of four colors, namely, the V-LED (Violet Light Emitting Diode) 32a, the B-LED (Blue Light Emitting Diode) 32b, a G-LED (Green Light Emitting Diode) 32c, and an R-LED (Red Light Emitting Diode) 32d. The V-LED 32a, the B-LED 32b, the G-LED 32c, and the R-LED 32d are semiconductor light sources that emit violet (V) light, blue (B) light, green (G) light, and red (R) light, which are observation light having a peak wavelength at, for example, 410 nm, 450 nm, 530 nm, and 615 nm, respectively.

The light source control unit 31 individually controls, for the respective LEDs, turning on or off of the four LEDs of the light source unit 32, the amount of light emitted at the time of turning on, and the like in accordance with the observation mode such as the normal-light observation mode and the special-light observation mode. In the normal-light observation mode, the light source control unit 31 turns on all of the V-LED 32a, the B-LED 32b, the G-LED 32c, and the R-LED 32d. In the normal-light observation mode, therefore, white light including V light, B light, G light, and R light is used as illumination light.

In the special-light observation mode, on the other hand, the light source control unit 31 turns on any one light source or an appropriate combination of a plurality of light sources among the V-LED 32a, the B-LED 32b, the G-LED 32c, and the R-LED 32d. In a case where a plurality of light sources are turned on, special light in which the amounts of light (the ratio of the amounts of light) to be emitted from the respective light sources are controlled is used as illumination light. This makes it possible to capture images of a plurality of layers having different depths of a photographic subject.

In this example, in the first observation mode, white light (WL) for a normal-light image is emitted. In the second observation mode, special light for a special-light image (BLI (Blue Light Imaging or Blue LASER Imaging), LCI (Linked Color Imaging), or NBI (Narrow Band Imaging)) is emitted.

The illumination light for BLI is illumination light having a high proportion of V light with high absorbance for the superficial blood vessel whereas the proportion of G light with high absorbance for the middle blood vessel is reduced, and is suitable for generating an image (BLI) suitable for enhancing a blood vessel or a structure in the mucosal superficial layer of a photographic subject.

The illumination light for LCI is illumination light in which the proportion of V light is higher than that of observation light for WL and which is more suitable for capturing a fine change in color tone than the observation light for WL, and is suitable for generating an image (LCI) subjected to color enhancement processing to make a reddish color more red and a whitish color more white relative to the color near the mucous membrane by also using the signal of the R component.

The illumination light for NBI is suitable for generating an image (NBI) in which a fine change in the surface to be irradiated is enhanced by narrowing the range of the wavelengths of illumination light to be applied.

Light of colors emitted from the LEDs 32a to 32d is incident on the light guide 40, which is inserted into the endoscope 11, via an optical path coupling portion formed by a dichroic mirror, a lens, and the like and an aperture diaphragm mechanism (not illustrated).

As the observation light of the light source device 12, light in various wavelength ranges according to an observation purpose is selected, such as white light (light in the white wavelength range or light in a plurality of wavelength ranges), light (special light) having a peak in one or a plurality of specific wavelength ranges, or a combination thereof.

A first example of the specific wavelength range is, for example, the blue range or the green range in the visible range. The wavelength range in the first example includes a wavelength range greater than or equal to 390 nm and less than or equal to 450 nm or greater than or equal to 530 nm and less than or equal to 550 nm, and light in the first example has a peak wavelength in the wavelength range greater than or equal to 390 nm and less than or equal to 450 nm or greater than or equal to 530 nm and less than or equal to 550 nm.

A second example of the specific wavelength range is, for example, the red range in the visible range. The wavelength range in the second example includes a wavelength range greater than or equal to 585 nm and less than or equal to 615 nm or greater than or equal to 610 nm and less than or equal to 730 nm, and light in the second example has a peak wavelength in the wavelength range greater than or equal to 585 nm and less than or equal to 615 nm or greater than or equal to 610 nm and less than or equal to 730 nm.

A third example of the specific wavelength range includes a wavelength range in which the absorption coefficient is different between oxyhemoglobin and reduced hemoglobin, and light in the third example has a peak wavelength in the wavelength range in which the absorption coefficient is different between oxyhemoglobin and reduced hemoglobin. The wavelength range in the third example includes a wavelength range of 400 ± 10 nm, 440 ± 10 nm, 470 ± 10 nm, or greater than or equal to 600 nm and less than or equal to 750 nm, and light in the third example has a peak wavelength in the wavelength range of 400 ± 10 nm, 440 ± 10 nm, 470 ± 10 nm, or greater than or equal to 600 nm and less than or equal to 750 nm described above.

A fourth example of the specific wavelength range is a wavelength range (390 nm to 470 nm) of excitation light that is used for observation (fluorescence observation) of fluorescence emitted from a fluorescent substance in a living body and that excites the fluorescent substance.

A fifth example of the specific wavelength range is the wavelength range of infrared light. The wavelength range in the fifth example includes a wavelength range greater than or equal to 790 nm and less than or equal to 820 nm or greater than or equal to 905 nm and less than or equal to 970 nm, and light in the fifth example has a peak wavelength in the wavelength range greater than or equal to 790 nm and less than or equal to 820 nm or greater than or equal to 905 nm and less than or equal to 970 nm.

The endoscope processor 13 has the processor operating unit 13a, the processor control unit 61, a ROM 62, a digital signal processor (DSP) 63, an image processing unit 65, a display control unit 66, a storage unit 67, and so on.

The processor operating unit 13a includes a power button, an input unit that accepts inputs such as a coordinate position pointed on the screen of the display device 14 by a mouse and a click (execution instruction), and so on.

The processor control unit 61 reads out a necessary program and data from the ROM 62 in accordance with the information on the operation performed by the processor operating unit 13a and information on the operation performed by the endoscope operating unit 46, which is received via the endoscope control unit 47, and sequentially processes the program and data to control the units of the endoscope processor 13 and control the light source device 12. The processor control unit 61 may accept a necessary instruction input from any other external device such as a keyboard connected via an interface (not illustrated).

Under the control of the processor control unit 61, the DSP 63 functioning as a form of image acquisition unit that acquires image data of each frame of a moving image output from the endoscope 11 (the imaging element 45) performs various types of signal processing, such as defect correction processing, offset processing, white balance correction, gamma correction, and demosaicing, on image data of one frame of the moving image input from the endoscope 11 to generate image data for the frame.

The image processing unit 65 receives image data from the DSP 63 and performs image processing, such as color conversion processing, color enhancement processing, and structure enhancement processing, on the received image data as necessary to generate image data indicating an endoscopic image in which an observation target appears. The color conversion processing is processing for performing color conversion on image data by using 3 × 3 matrix processing, gradation transformation processing, three-dimensional look-up table processing, and so on. The color enhancement processing is processing for color enhancement for image data subjected to color conversion processing, for example, in a direction of making a difference in tint between a blood vessel and a mucous membrane. The structure enhancement processing is, for example, processing for enhancing a specific tissue or structure included in an observation target such as a blood vessel or a pit pattern and is performed on image data after color enhancement processing.

The image data of each frame of the moving image processed by the image processing unit 65 is recorded in the storage unit 67 as a still image or a moving image instructed to be captured when an instruction is given to capture a still image or a moving image.

The display control unit 66 generates display data for displaying a normal-light image or a special-light image on the display device 14 on the basis of the image data input from the image processing unit 65, outputs the generated display data to the display device 14, and causes the display device 14 to display a display image (such as a moving image captured by the endoscope 11).

Further, the display control unit 66 causes the display device 14 to display a recognition result input from the detector 15 via the image processing unit 65 or a recognition result input from the detector 15.

When a region of interest is detected by the detector 15, the display control unit 66 displays an index indicating the region of interest so as to be superimposed on an image displayed on the display device 14. Examples of the index include highlighting such as changing the color of the region of interest in the display image, displaying a marker, and displaying a bounding box.

Further, the display control unit 66 can display, based on the detection result of the detection target by the detector 15, information indicating the presence or absence of the detection target so as not to overlap with the image displayed on the display device 14. The information indicating the presence or absence of the detection target may be, for example, such that the color of the frame of the endoscopic image is changed between when the detection target is detected and when the detection target is not detected, or such that the text "the detection target is present!" is displayed in a display region different from the endoscopic image.

When the detector 15 performs discrimination for a lesion, the display control unit 66 causes the display device 14 to display the discrimination result. Examples of the method for displaying the discrimination result include displaying text indicating the discrimination result in a display image on the display device 14. The text may not necessarily be displayed in the display image, and may be displayed in any way so long as the correspondence relationship with the display image can be identified.

### [Detector 15]

Next, the detector 15 according to the present invention will be described.

The detector 15 is a section that detects a detection target such as a lesion from images sequentially captured by an imaging unit (the endoscope 11), and sequentially accepts images subjected to image processing by the endoscope processor 13. In this example, in the first observation mode, a normal-light image (WL image) is accepted as an image for detection. In the second observation mode, a special-light image (BLI, CLI, or NBI) is accepted as an image for detection.

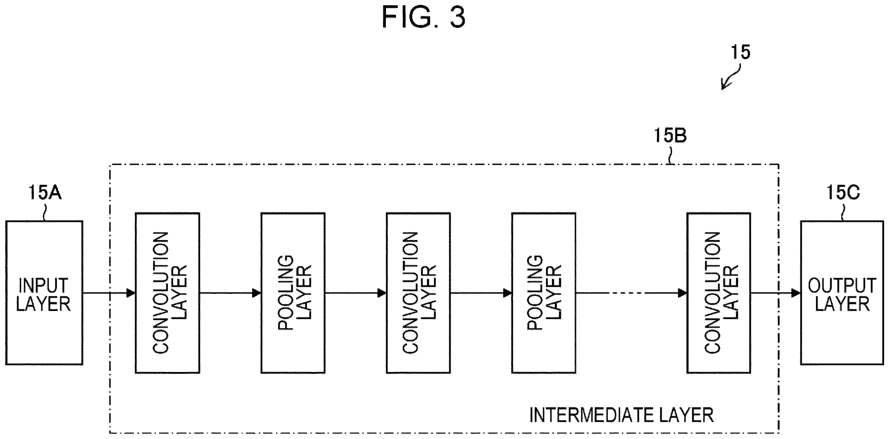

Fig. 3 is a schematic diagram illustrating a typical example configuration of a convolutional neural network (CNN), which is one of the learning models constituting the detector 15.

A CNN 15 is, for example, a learning model for detecting the position of a detection target (such as a lesion, a scar from an operation, or a scar after a treatment) appearing in an endoscopic image or discriminating the type of the lesion. The CNN 15 has a multiple-layer structure and holds a plurality of weight parameters. The weight parameters are set to optimum values, thereby allowing the CNN 15 to become a learned model and function as a detector.

As illustrated in Fig. 3, the CNN 15 includes an input layer 15A, an intermediate layer 15B having a plurality of convolution layers and a plurality of pooling layers, and an output layer 15C, and each layer has a structure in which a plurality of "nodes" are coupled using "edges".

In this example, the CNN 15 is a learning model that performs segmentation for recognizing the position of the detection target appearing in the endoscopic image. The learning model to which a fully convolutional network (FCN: Fully Convolutional Network), which is a type of CNN, is applied to the CNN 15. Examples of the FCN includes: one that determines the position of the detection target appearing in the endoscopic image on a pixel-by-pixel basis or determines the presence or absence of the detection target in units of several pixels; one that outputs the values of the coordinates of the center of the detection target, the values of the coordinates of four corners of a rectangular shape surrounding the detection target, and the like.

An image of one frame for detection is input to the input layer 15A.

The intermediate layer 15B is a portion that extracts a feature from an image input from the input layer 15A. Each of the convolution layers of the intermediate layer 15B performs filtering processing (performs a convolution operation using a filter) on an image or a nearby node in the preceding layer to acquire a "feature map". The pooling layers reduce (or enlarge) the feature maps output from the convolution layers to obtain new feature maps. The "convolution layer" plays a role of feature extraction such as edge extraction from an image, and the "pooling layer" plays a role of providing robustness so that the extracted features are not affected by parallel displacement or the like. The intermediate layer 15B does not necessarily include sets each including a convolution layer and a pooling layer, and may be configured such that convolution layers are consecutive, or may also include a normalization layer.

The output layer 15C is a portion that outputs a detection result obtained by detecting the position of the detection target appearing in the endoscopic image or classifying (discriminating) the type of the lesion on the basis of the features extracted by the intermediate layer 15B.

The CNN 15 is learned using a large number of sets each including an image set for learning and correct answer data for the image set, and filter coefficients or offset values to be applied to the respective convolution layers of the CNN 15 are set to optimum values by using data sets for learning. The correct answer data is preferably a discrimination result or a detection target designated by a doctor for the endoscopic image.

In this example, the CNN 15 is configured to recognize the position of the detection target appearing in the endoscopic image. However, the detector (CNN) is not limited to this, and may be configured to execute discrimination for the lesion and output a discrimination result. For example, the detector may classify the endoscopic image into three categories including "neoplastic", "non-neoplastic", and "other" and output three scores corresponding to "neoplastic", "non-neoplastic", and "other" (the total of the three scores is 100%) as the discrimination result, or may output the classification result if the endoscopic image can be clearly classified from the three scores. In addition, a CNN that outputs such a discrimination result preferably has a fully connected layer as the last one layer or a plurality of layers of the intermediate layer instead of the fully convolutional network (FCN).

Furthermore, the detector 15 preferably uses a learning model learned using a normal-light image when a normal-light image is to be input, and applies a learning model learned using a special-light image when a special-light image is to be input.

### [Non-claimed First Embodiment]

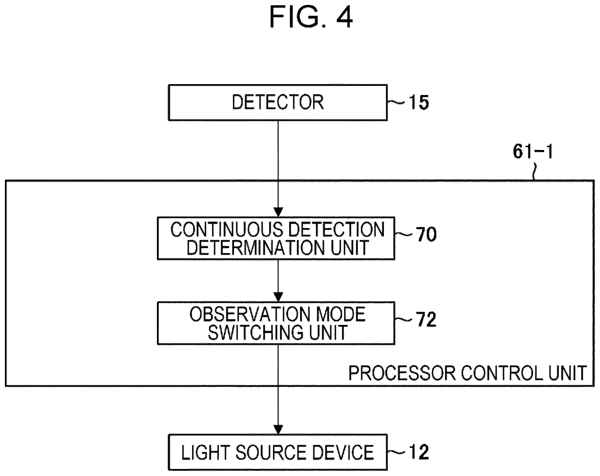

Fig. 4 is a block diagram illustrating a main part of a first embodiment of an endoscope processor in an endoscope apparatus according to the present invention.

The endoscope processor 13 of the first embodiment illustrated in Fig. 4 includes a processor control unit 61-1.

The processor control unit 61-1 is a section that performs overall control of the units of the endoscope processor 13. The processor control unit 61-1 of the first embodiment further includes a continuous detection determination unit 70 and an observation mode switching unit 72.

The continuous detection determination unit 70 receives a detection result from the detector 15 and determines whether the detector 15 continuously detects the detection target on the basis of the received detection result. The determination of whether the detection target has been detected from one frame (image) can be performed, for example, based on whether a pixel having the detection target is present in a case where the detector 15 outputs the result of the determination of the presence or absence of the detection target on a pixel-by-pixel basis or in units of several pixels, or can be performed based on whether the values of the coordinates are output in a case where the detector 15 outputs the values of the coordinates of the center of the detection target or the values of the coordinates of four corners of a rectangular shape surrounding the detection target.

The continuous detection determination unit 70 can determine that the detector 15 continuously detects the detection target when the detector 15 detects the detection target consecutively within a certain time range longer than the detection interval of the detector 15 (the period of one frame of the moving image or the period of a plurality of frames of the moving image).

Whether the detection target is continuously detected is considered to be determined by sequentially storing detection results of the detector 15 and referring to the current detection result and the most recent detection result.

Preferably, the continuous detection determination unit 70 determines that the detector 15 continuously detects the detection target not only when the detector 15 detects the detection target at each detection interval within a certain time range longer the detection interval of the detector 15 but also when the detector 15 detects the detection target at a rate equal to or higher than a threshold value.

For example, when the detector 15 detects the detection target in the period of one frame of the moving image (for each frame), it is considered to refer to the detection results for the most recent 60 frames (for one second) that are sequentially input. In the determination of continuous detection, it may be determined that the detection target is continuously detected not only when, for example, the detection target is detected in all of 60 frames in a case where the previous 60 frames are referred to but also when, for example, the detection target is detected in every other frame to every three frames.

The observation mode switching unit 72 is a section that switches between the first observation mode and the second observation mode. In a state where the first observation mode is used, the observation mode switching unit 72 automatically switches from the first observation mode to the second observation mode when the continuous detection determination unit 70 determines that the detection target is continuously detected.

In this example, the first observation mode is a normal-light observation mode in which WL (normal light) is emitted for observation, and the second observation mode is a special-light observation mode in which special light for a special-light image (BLI, LCI, or NBI) is emitted for observation.

Accordingly, the observation mode switching unit 72 outputs a command for switching from the first observation mode to the second observation mode or a command for switching from emission of WL to emission of special light to the light source device 12 to automatically switch from the first observation mode to the second observation mode.

When a certain period of time (for example, several seconds) has elapsed after the switching of the observation mode to the second observation mode, the observation mode switching unit 72 switches to the first observation mode. Alternatively, after switching to the second observation mode, the observation mode switching unit 72 may switch to the first observation mode when the continuous detection determination unit 70 determines that the detection target is not continuously detected.

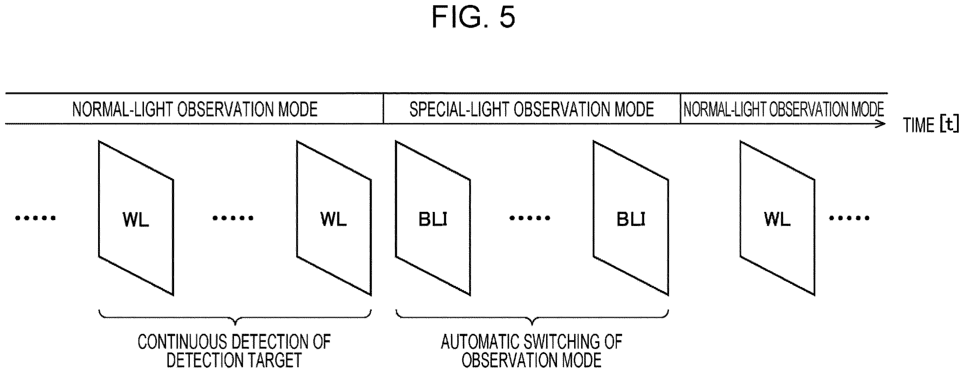

Fig. 5 is a conceptual diagram illustrating automatic switching of the observation mode when a normal-light observation mode using WL is set as the first observation mode and a special-light observation mode using special light for BLI is set as the second observation mode.

As illustrated in Fig. 5, when the detection target is continuously detected from normal-light images (WL images) sequentially captured in the normal-light observation mode, the observation mode switching unit 72 automatically switches from the normal-light observation mode to the special-light observation mode, and special-light images (BLI) are captured in the special-light observation mode. When a certain period of time has elapsed after the switching of the observation mode to the special-light observation mode, the observation mode is switched again to the normal-light observation mode by the observation mode switching unit 72.

Accordingly, in a state where the normal-light observation mode is used, the observation mode is automatically switched from the normal-light observation mode to the special-light observation mode when the detection target is continuously detected from sequentially captured images. Thus, it is possible to capture an image of the detection target under special light suitable for detailed observation of the detection target, and to reduce the burden of the operator's operation of switching the observation mode.

### [Second Embodiment]

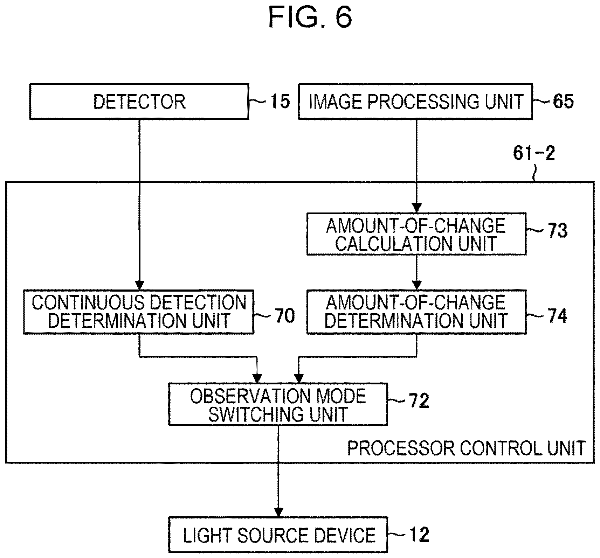

Fig. 6 is a block diagram illustrating a main part of a second embodiment of an endoscope processor in an endoscope apparatus according to the present invention. In Fig. 6, components common to those of the first embodiment illustrated in Fig. 4 are denoted by the same reference numerals, and detailed description thereof will be omitted.

The endoscope processor 13 of the second embodiment illustrated in Fig. 6 includes a processor control unit 61-2.

The processor control unit 61-2 is different from the processor control unit 61-1 illustrated in Fig. 4 mainly in that an amount-of-change calculation unit 73 and an amount-of-change determination unit 74 are added.

The amount-of-change calculation unit 73 sequentially receives image data of the respective frames of the moving image processed by the image processing unit 65 and computes an amount of change in a specific region of images captured by the endoscope 11 on the basis of the sequentially received image data.

The specific region of the images can be the entire region of a captured image. The amount of change in the specific region can be the amount of change in the size or position of the consecutively captured images.

The amount-of-change determination unit 74 determines whether the amount of change calculated by the amount-of-change calculation unit 73 is within a threshold value.

For example, when observation is performed while the endoscope insertion section is pulled out from the body cavity, the image being observed is varying, whereas when the endoscope insertion section is temporarily stopped from being pulled out, the image being observed is stationary. Accordingly, the threshold value for the amount of change can be a value set as a criterion for determining whether the size or position of the specific region between consecutive images has changed with the movement of the endoscope insertion section.

In a state where the normal-light observation mode is used, the observation mode switching unit 72 automatically switches from the normal-light observation mode to the special-light observation mode when the continuous detection determination unit 70 determines that the detection target is continuously detected and the amount-of-change determination unit 74 determines that the amount of change in a specific region of the images is within the threshold value.

After switching to the special-light observation mode, the observation mode switching unit 72 switches from the special-light observation mode to the normal-light observation mode when the amount-of-change determination unit 74 determines that the amount of change in a specific region of the images is larger than the threshold value.

According to the second embodiment, in a state where the normal-light observation mode is used, when a detection target is continuously detected from sequentially captured images and the amount-of-change determination unit 74 determines that the amount of change in the specific region is small (when the amount of change is determined to be within the threshold value), it is considered that the endoscope insertion section remains substantially stationary (the operator is gazing at the detection target). Thus, the observation mode is automatically switched from the normal-light observation mode to the special-light observation mode.

In contrast, even when the detection target is continuously detected from sequentially captured images, if the amount-of-change determination unit 74 determines that the amount of change in specific region is large (if the amount of change is determined to be larger than the threshold value), it is considered that the endoscope insertion section is moving (for example, observation is being performed while the endoscope insertion section is pulled out from the body cavity). Thus, the observation mode is not switched from the normal-light observation mode to the special-light observation mode.

The specific region is not limited to the entire region of a captured image, and may be, for example, a center region of a captured image.

### [Third Embodiment]

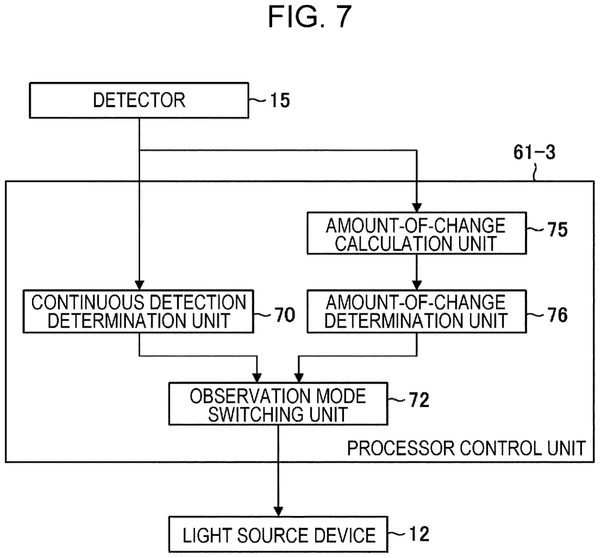

Fig. 7 is a block diagram illustrating a main part of a third embodiment of an endoscope processor in an endoscope apparatus according to the present invention. In Fig. 7, components common to those of the second embodiment illustrated in Fig. 6 are denoted by the same reference numerals, and detailed description thereof will be omitted.

The endoscope processor 13 of the third embodiment illustrated in Fig. 7 includes a processor control unit 61-3.

The processor control unit 61-3 is different from the processor control unit 61-2 illustrated in Fig. 6 mainly in the target for which the amount of change is determined by an amount-of-change calculation unit 75 and the content of determination of the amount of change by an amount-of-change determination unit 76.

The amount-of-change calculation unit 75 sequentially receives detection information of the detection target detected by the detector 15 and computes an amount of change in the detection target on the basis of the sequentially received detection information of the detection target. That is, the amount-of-change calculation unit 75 sets the detection target calculated based on the detection information from the detector 15 as a specific region and computes an amount of change in the region (specific region) corresponding to the detection target.

The amount of change in the detection target computed by the amount-of-change calculation unit 75 can be the amount of change in the position of the detection target that is consecutively detected.

When the detection information obtained by the detector 15 is, for example, the center coordinates of the detection target (lesion area) (the center coordinates can be computed regardless of whether the detection result is the presence or absence of a lesion in units of pixels or the coordinates of a rectangular shape), the coordinates in the current frame and the coordinates in the preceding frame are compared, and the amount of change is computed.

The amount-of-change determination unit 76 determines whether the amount of change in the detection target computed by the amount-of-change calculation unit 75 is within a threshold value. For example, when the detector 15 continuously detects the detection target and the amount of change in the center coordinates of the detection target is within 32 pixels, the amount-of-change determination unit 76 can determine that the amount of change in the detection target is within the threshold value.

In a state where the normal-light observation mode is used, the observation mode switching unit 72 automatically switches from the normal-light observation mode to the special-light observation mode when the continuous detection determination unit 70 determines that the detection target is continuously detected and the amount-of-change determination unit 76 determines that the amount of change in the detection target is within the threshold value.

After switching to the special-light observation mode, the observation mode switching unit 72 switches from the special-light observation mode to the normal-light observation mode when the amount-of-change determination unit 76 determines that the amount of change in the detection target is larger than the threshold value.

The amount of change in the detection target is not limited to the amount of change in the position (center coordinates) of the detection target, and may be the amount of change in the size of the detection target. For example, when the detection result obtained by the detector 15 is, for example, the values of the coordinates of four corners of a rectangular shape surrounding the detection target (lesion area), the area of the rectangular shape in the current frame and the area of the rectangular shape in the preceding frame are compared, and the amount of change is computed. For example, when the detector 15 continuously detects the detection target and the change in area is within 80% of that of the preceding frame, the amount of change in the detection target can be determined to be within the threshold value.

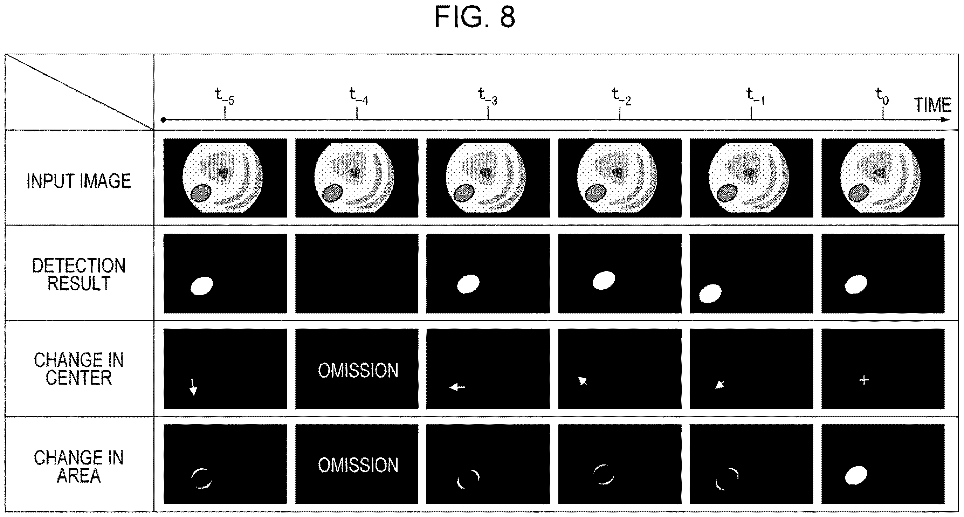

Fig. 8 is a diagram illustrating input images (frames) sequentially captured by the endoscope 11, detection results of the detection target detected from the input images, amounts of change in the center coordinates of the detection target, and amounts of change in the size (area) of the detection target.

In Fig. 8, a frame at time t₀ is the current frame, and frames at time t₋₁ to time t₋₅ are previous frames.

The detector 15 detects the detection target from an input frame. In Fig. 8, the detection target is not detected in the frame at time t₋₄, and the detection target is detected in the frames at the other times.

In Fig. 8, an amount of change in the center coordinates of the detection target between consecutive frames is indicated by a vector (arrow), and an amount of change in the area of the detection target between consecutive frames is indicated by crescent-shaped regions. The amount of change in the detection target between consecutive frames includes: an amount of change between a detection region in which the detection target exists in the subsequent frame but the detection target does not exist in the preceding frame; and a detection region in which the detection target does not exist in the subsequent frame but the detection target exists in the preceding frame.

The amount-of-change calculation unit 75 illustrated in Fig. 7 computes the amount of change in the center coordinates of the detection target indicated by the vector or computes the area of the crescent-shaped regions, which is the amount of change in the area of the detection target. The amount-of-change determination unit 76 determines whether the amount of change in the center coordinates of the detection target or the amount of change in the area of the detection target computed by the amount-of-change calculation unit 75 is within a threshold value.

Since the detection target is not detected in the frame at time t₋₄ and the detection target is detected in the frames at the other times, the continuous detection determination unit 70 can determine that the detection target is continuously detected.

### [Other Embodiment of Switching of Observation Mode]

In the embodiments described above, the first observation mode is the normal-light observation mode, and the second observation mode is the special-light observation mode. However, the present invention is not limited to this. For example, the first observation mode may be a first special-light observation mode, and the second observation mode may be a second special-light observation mode.

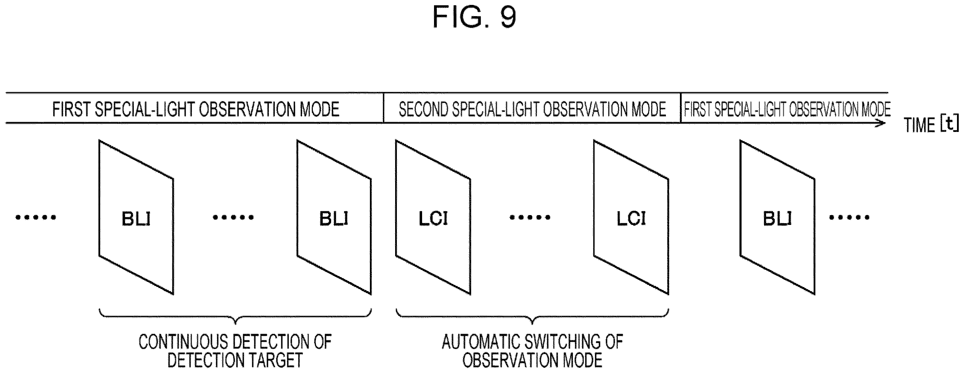

Fig. 9 is a conceptual diagram illustrating automatic switching of the observation mode when a first special-light observation mode using special light for BLI is set as the first observation mode and a second special-light observation mode using special light for LCI is set as the second observation mode.

As illustrated in Fig. 9, when the detection target is continuously detected from first special-light images (BLI) sequentially captured in the first special-light observation mode, the observation mode switching unit 72 automatically switches from the first special-light observation mode to the second special-light observation mode, and second special-light images (LCI) are captured in the second special-light observation mode. When a certain period of time has elapsed after the switching of the observation mode to the second special-light observation mode, the observation mode is switched again to the first special-light observation mode by the observation mode switching unit 72.

Accordingly, in a state where the first special-light observation mode is used, the observation mode is automatically switched from the first special-light observation mode to the second special-light observation mode when the detection target is continuously detected from sequentially captured images. Thus, it is possible to capture an image of the detection target in LCI having a different feature from BLI, and to reduce the burden of the operator's operation of switching the observation mode.

### [Method for Operating Endoscope Apparatus]

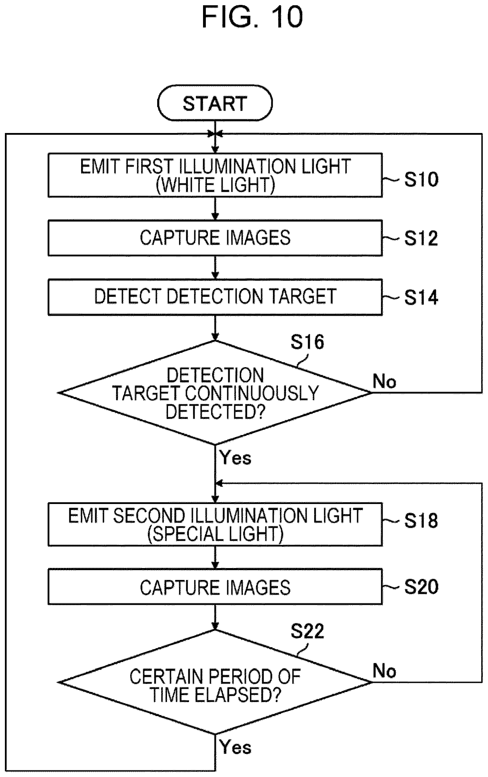

Fig. 10 is a flowchart illustrating an embodiment of a method for operating an endoscope apparatus according to the present invention and illustrates the processing procedures of the respective units of the endoscope apparatus 10 illustrated in Fig. 2.

In Fig. 10, first, the observation mode is set to the first observation mode, and the light source device 12, which is controlled by the processor control unit 61, emits white light as first illumination light (step S10). The endoscope 11 sequentially captures images (WL images) of the photographic subject irradiated with white light (WL) (step S12).

The detector 15 detects the detection target from the WL images captured by the endoscope 11 (step S14).

The continuous detection determination unit 70 (Fig. 4) determines whether the detection target is continuously detected by the detector 15 (step S16). If it is determined that the detection target is not continuously detected (in the case of "No"), the process returns to step S10, and the processing of steps S10 to S16 is repeatedly performed.

On the other hand, if it is determined that the detection target is continuously detected (in the case of "Yes"), a transition to step S18 occurs. That is, in a state where the first observation mode is used, if it is determined that the detection target is continuously detected, the observation mode switching unit 72 switches to the second observation mode in which second illumination light (special light) is emitted.

In step S18, special light is emitted from the light source device 12. The endoscope 11 sequentially captures images (special-light images) of the photographic subject irradiated with special light (step S20).

The processor control unit 61 determines whether a certain period of time has elapsed after the switching of the observation mode to the second observation mode (step S22). If it is determined that the certain period of time has not elapsed (in the case of "No"), a transition to step S18 occurs, and special-light images are continuously captured.

On the other hand, if it is determined that the certain period of time has elapsed (in the case of "Yes"), a transition to step S10 occurs. Accordingly, the observation mode is returned from the second observation mode to the first observation mode, and WL images are captured again.

### [Other]

In this embodiment, when imaging in the second observation mode continues for a certain period of time or when the detection target is not continuously detected after the observation mode is automatically switched from the first observation mode to the second observation mode, the observation mode is switched again to the first observation mode. However, this is not limiting, and when a still image is captured and recorded in accordance with the operation of the still-image capturing button after the observation mode is automatically switched from the first observation mode to the second observation mode, the observation mode may be switched to the first observation mode.

Alternatively, when the observation mode is automatically switched from the first observation mode to the second observation mode, a still image may be automatically captured and recorded, and, after that, the observation mode may be switched to the first observation mode. This makes it possible to automatically switch the observation mode and to automatically capture a still image. The burden of the operator's operation of the endoscope can further be reduced. In this case, in the second observation mode, the display device 14 or the like preferably notifies that a still image has been captured.

In this embodiment, furthermore, observation modes corresponding to different types of illumination light (illumination light for WL, BLI, LCI, and NBI) have been described. However, the observation modes are not limited to those corresponding to these types of illumination light. It is possible to appropriately set which of the two or more types of observation modes is set as each of the first observation mode and the second observation mode.

In this embodiment, furthermore, the endoscope apparatus 10 including the endoscope 11, the light source device 12, the endoscope processor 13, and the detector 15 has been described. However, the present invention is not limited to the endoscope apparatus 10, and may be implemented as the endoscope processor 13 not including the endoscope 11 as an element. In this case, the endoscope processor 13, the light source device 12, and the detector 15 may be integrated or separated.

In addition, the different types of illumination light are not limited to light emitted from LEDs of four colors. For example, a blue laser diode that emits blue laser light having a center wavelength of 445 nm, and a bluish violet laser diode that emits bluish violet laser light having a center wavelength of 405 nm may be used as light-emitting sources, and a YAG (Yttrium Aluminum Garnet) based fluorescent body may be irradiated with laser light of the blue laser diode and laser light of the bluish violet laser diode to emit light. When the fluorescent body is irradiated with blue laser light, the fluorescent body is excited to emit broadband fluorescent light, and a portion of the blue laser light passes through the fluorescent body as it is. The bluish violet laser light is transmitted without exciting the fluorescent body. Accordingly, adjusting the intensities of the blue laser light and the bluish violet laser light makes it possible to emit illumination light for WL, illumination light for BLI, and illumination light for LCI. In addition, emitting only the bluish violet laser light makes it possible to emit observation light having a center wavelength of 405 nm.

Furthermore, the present invention is also applicable to an endoscope apparatus including an endoscope (imaging unit) including a monochrome imaging element having no color filter, instead of the imaging element 45, which is a color imaging element. When a normal-light image or a special-light image, which is a color endoscopic image, is to be acquired using the monochrome imaging element, the subject is sequentially illuminated with illumination light of different colors, and an image is captured for each illumination light (images are captured in a frame sequential manner).

For example, illumination light of different colors (R light, G light, B light, or V light) is sequentially emitted from the light source unit 32, thereby capturing an R image, a G image, a B image, or a V image of a color corresponding to the R light, the G light, the B light, or the V light in a frame sequential manner by using the monochrome imaging element.

The endoscope processor can generate an image (for example, a WL image, BLI, LCI, NBI, or the like) corresponding to the first observation mode or the second observation mode from one or a plurality of images (an R image, a G image, a B image, or a V image) captured in a frame sequential manner. The image according to the observation mode, such as a WL image, BLI, LCI, or NBI, can be generated by adjusting the combination ratio of a plurality of images captured in a frame sequential manner. In the present invention, also in a case where images are captured in a frame sequential manner to generate, in accordance with an observation mode, images corresponding to the observation mode, the images are included in images of the photographic subject irradiated with illumination light emitted from a light source unit in accordance with the observation mode.

In addition, the detector 15 is not limited to a CNN, and may be, for example, a machine learning model other than a CNN, such as DBN (Deep Belief Network) or SVM (Support Vector Machine). That is, the detector 15 may be any device capable of detecting a detection target from an image.

Further, the hardware structure of the endoscope processor 13 and/or the detector 15 is implemented as the following various processors. The various processors include a CPU (Central Processing Unit), which is a general-purpose processor executing software (program) to function as various control units, a programmable logic device (PLD) such as an FPGA (Field Programmable Gate Array), which is a processor whose circuit configuration is changeable after manufacture, a dedicated electric circuit, which is a processor having a circuit configuration specifically designed to cause specific processing to be executed, such as an ASIC (Application Specific Integrated Circuit), and so on.

A single processing unit may be configured as one of the various processors or as a combination of two or more processors of the same type or different types (for example, a plurality of FPGAs or a combination of a CPU and an FPGA). Alternatively, a plurality of control units may be configured as a single processor. Examples of the configuration of a plurality of control units as a single processor include, first, a form in which, as typified by a computer such as a client or a server, the single processor is configured as a combination of one or more CPUs and software and the processor functions as the plurality of control units. The examples include, second, a form in which, as typified by a system on chip (SoC) or the like, a processor is used in which the functions of the entire system including the plurality of control units are implemented as one IC (Integrated Circuit) chip. As described above, the various control units are configured by using one or more of the various processors described above as a hardware structure.

Moreover, it is needless to say that the present invention is not limited to the embodiments described above and various modifications may be made without departing from the scope of the present invention.

- 10: endoscope apparatus
- 11: endoscope
- 12: light source device
- 12a: light source operating unit
- 13: endoscope processor
- 13a: processor operating unit
- 14: display device
- 15: detector
- 15A: input layer
- 15B: intermediate layer
- 15C: output layer
- 16: insertion section
- 16a: insertion section distal end
- 16b: bending portion
- 16c: flexible pipe portion
- 17: handheld operation section
- 18: universal cord
- 21: angle knob
- 22: operation button
- 23: forceps inlet
- 25a, 25b: connector portion
- 31: light source control unit
- 32: light source unit
- 32a: V-LED
- 32b: B-LED
- 32c: G-LED
- 32d: R-LED
- 40: light guide
- 42: illumination lens
- 44: objective lens
- 45: imaging element
- 46: endoscope operating unit
- 47: endoscope control unit
- 48: ROM
- 61, 61-1, 61-2, 61-3: processor control unit
- 62: ROM
- 65: image processing unit
- 66: display control unit
- 67: storage unit
- 70: continuous detection determination unit
- 72: observation mode switching unit
- 73, 75: amount-of-change calculation unit
- 74, 76: amount-of-change determination unit
- S10 to S22: step

## Claims

1. An endoscope apparatus comprising:
a light source unit (12) configured to emit first illumination light and second illumination light respectively corresponding to a first observation mode and a second observation mode;
an imaging unit (11) configured to capture an image of a photographic subject irradiated with the first illumination light or the second illumination light;
a detector (15) configured to detect a detection target from images sequentially captured by the imaging unit (11);
a continuous detection determination unit (70) configured to determine whether the detector (15) continuously detects the detection target; and
an observation mode switching unit (72) configured to switch between the first observation mode and the second observation mode;
an amount-of-change calculation unit (73) configured to calculate an amount of change in a specific region of images captured by the imaging unit (11); and
an amount-of-change determination unit (74) configured to determine whether the amount of change calculated by the amount-of-change calculation unit (73) is within a threshold value,
wherein in a state where the first observation mode is used, the observation mode switching unit (72) is configured to automatically switch to the second observation mode in response to the continuous detection determination unit (70) determining that the detection target is continuously detected and the amount-of-change determination unit (74) determining that the amount of change is within the threshold value, the combination of both indicating that the endoscope insertion section remains substantially stationary.

2. The endoscope apparatus according to claim 1, wherein the specific region is an entire region of an image captured by the imaging unit (11).

3. The endoscope apparatus according to claim 1, wherein the specific region is a center region of an image captured by the imaging unit (11).

4. The endoscope apparatus according to claim 1, wherein the specific region is a region corresponding to the detection target, the region being calculated based on detection information from the detector (15).

5. The endoscope apparatus according to any one of claims 1 to 4, wherein the amount of change calculated by the amount-of-change calculation unit (73) is an amount of change in a position of the specific region.

6. The endoscope apparatus according to any one of claims 1 to 4, wherein the amount of change calculated by the amount-of-change calculation unit (73) is an amount of change in a size of the specific region.

7. The endoscope apparatus according to any one of claims 1 to 6, wherein in response to an elapse of a certain period of time after the observation mode switching unit (72) switches to the second observation mode, the observation mode switching unit (72) is configured to switch to the first observation mode.

8. The endoscope apparatus according to any one of claims 1 to 6, wherein after the observation mode switching unit (72) switches to the second observation mode, the observation mode switching unit (72) is configured to switch to the first observation mode in response to the continuous detection determination unit (70) determining that the detection target is not continuously detected.

9. The endoscope apparatus according to any one of claims 1 to 6, wherein after the observation mode switching unit (72) switches to the second observation mode, the observation mode switching unit (72) is configured to switch to the first observation mode in response to the amount-of-change determination unit (74) determining that the amount of change is larger than the threshold value and determining that the endoscope insertion section remains substantially stationary.

10. The endoscope apparatus according to any one of claims 1 to 6, wherein after the observation mode switching unit (72) switches to the second observation mode, the observation mode switching unit (72) is configured to switch to the first observation mode in response to a still image being captured.

11. The endoscope apparatus according to any one of claims 1 to 10, wherein the continuous detection determination unit (70) is configured to determine that the detector (15) continuously detects the detection target in response to the detector (15) consecutively detecting the detection target within a certain time range longer than a detection interval of the detector (15).

12. The endoscope apparatus according to any one of claims 1 to 11, wherein the continuous detection determination unit (70) is configured to determine that the detector (15) continuously detects the detection target in response to the detector (15) detecting the detection target at a rate greater than or equal to a threshold value within a certain time range longer than a detection interval of the detector (15).

13. The endoscope apparatus according to any one of claims 1 to 12, wherein the first observation mode is a normal-light observation mode in which normal light is used as the first illumination light, and the second observation mode is a special-light observation mode in which special light is used as the second illumination light.

14. The endoscope apparatus according to any one of claims 1 to 12, wherein the first observation mode is a first special-light observation mode in which first special light is used as the first illumination light, and the second observation mode is a second special-light observation mode in which second special light different from the first special light is used as the second illumination light.

## Patentansprüche

1. Endoskopvorrichtung, aufweisend:
eine Lichtquelleneinheit (12), die so konfiguriert ist, dass sie ein erstes Beleuchtungslicht und ein zweites Beleuchtungslicht aussendet, die jeweils einem ersten Beobachtungsmodus und einem zweiten Beobachtungsmodus entsprechen;
eine Abbildungseinheit (11), die so konfiguriert ist, dass sie ein Bild eines fotografischen Objekts aufnimmt, das mit dem ersten Beleuchtungslicht oder dem zweiten Beleuchtungslicht bestrahlt wird;
einen Detektor (15), der so konfiguriert ist, dass er ein Erfassungsziel aus den von der Abbildungseinheit (11) sequenziell erfassten Bildern erkennt;
eine Einheit (70) zur Bestimmung der kontinuierlichen Erfassung, die so konfiguriert ist, dass sie bestimmt, ob der Detektor (15) das Erfassungsziel kontinuierlich erfasst; und
eine Beobachtungsmodus-Umschalteinheit (72), die zum Umschalten zwischen dem ersten Beobachtungsmodus und dem zweiten Beobachtungsmodus konfiguriert ist;
eine Änderungsbetragsberechnungseinheit (73), die so konfiguriert ist, dass sie einen Änderungsbetrag in einem spezifischen Bereich von durch die Bildgebungseinheit (11) erfassten Bildern berechnet; und
eine Änderungsbetragsbestimmungseinheit (74), die so konfiguriert ist, dass sie bestimmt, ob der von der Änderungsbetragsberechnungseinheit (73) berechnete Änderungsbetrag innerhalb eines Schwellenwerts liegt,
wobei in einem Zustand, in dem der erste Beobachtungsmodus verwendet wird, die Beobachtungsmodus-Umschalteinheit (72) so konfiguriert ist, dass sie automatisch in den zweiten Beobachtungsmodus umschaltet, wenn die Einheit (70) zur Bestimmung der kontinuierlichen Erfassung feststellt, dass das Erfassungsziel kontinuierlich erfasst wird, und die Änderungsbetragsbestimmungseinheit (74) feststellt, dass der Änderungsbetrag innerhalb des Schwellenwerts liegt, wobei die Kombination von beidem anzeigt, dass der Endoskop-Einführabschnitt im Wesentlichen stationär bleibt.

2. Endoskopgerät nach Anspruch 1, wobei der spezifische Bereich ein ganzer Bereich eines von der Abbildungseinheit (11) aufgenommenen Bildes ist.

3. Endoskopgerät nach Anspruch 1, wobei der spezifische Bereich ein zentraler Bereich eines von der Abbildungseinheit (11) aufgenommenen Bildes ist.

4. Endoskopgerät nach Anspruch 1, wobei der spezifische Bereich ein Bereich ist, der dem Erfassungsziel entspricht, wobei der Bereich auf der Grundlage von Erfassungsinformationen vom Detektor (15) berechnet wird.

5. Endoskopgerät nach einem der Ansprüche 1 bis 4, wobei der von der Änderungsbetragsberechnungseinheit (73) berechnete Änderungsbetrag ein Änderungsbetrag in einer Position des spezifischen Bereichs ist.

6. Endoskopgerät nach einem der Ansprüche 1 bis 4, wobei der von der Änderungsbetragsberechnungseinheit (73) berechnete Änderungsbetrag ein Änderungsbetrag in einer Größe des spezifischen Bereichs ist.

7. Endoskopgerät nach einem der Ansprüche 1 bis 6, wobei die Beobachtungsmodus-Umschalteinheit (72) so konfiguriert ist, dass sie nach Ablauf einer bestimmten Zeitspanne, nachdem die Beobachtungsmodus-Umschalteinheit (72) auf den zweiten Beobachtungsmodus umgeschaltet hat, auf den ersten Beobachtungsmodus umschaltet.

8. Endoskopvorrichtung nach einem der Ansprüche 1 bis 6, wobei nach dem Umschalten der Beobachtungsmodus-Umschalteinheit (72) in den zweiten Beobachtungsmodus die Beobachtungsmodus-Umschalteinheit (72) so konfiguriert ist, dass sie in den ersten Beobachtungsmodus umschaltet, wenn die Einheit (70) zur Bestimmung der kontinuierlichen Erfassung feststellt, dass das Erfassungsziel nicht kontinuierlich erfasst wird.

9. Endoskopvorrichtung nach einem der Ansprüche 1 bis 6, wobei nach dem Umschalten der Beobachtungsmodus-Umschalteinheit (72) in den zweiten Beobachtungsmodus die Beobachtungsmodus-Umschalteinheit (72) so konfiguriert ist, dass sie in den ersten Beobachtungsmodus umschaltet, wenn die Änderungsbetragsbestimmungseinheit (74) feststellt, dass der Änderungsbetrag größer als der Schwellenwert ist, und feststellt, dass der Endoskop-Einführabschnitt im Wesentlichen stationär bleibt.

10. Endoskopgerät nach einem der Ansprüche 1 bis 6, wobei nach dem Umschalten der Beobachtungsmodus-Umschalteinheit (72) in den zweiten Beobachtungsmodus die Beobachtungsmodus-Umschalteinheit (72) so konfiguriert ist, dass sie als Reaktion auf die Aufnahme eines Standbildes in den ersten Beobachtungsmodus umschaltet.

11. Endoskopvorrichtung nach einem der Ansprüche 1 bis 10, wobei die Einheit (70) zur Bestimmung der kontinuierlichen Erfassung so konfiguriert ist, dass sie bestimmt, dass der Detektor (15) das Erfassungsziel kontinuierlich erfasst, wenn der Detektor (15) das Erfassungsziel innerhalb eines bestimmten Zeitbereichs, der länger ist als ein Erfassungsintervall des Detektors (15), nacheinander erfasst.

12. Endoskopvorrichtung nach einem der Ansprüche 1 bis 11, wobei die Einheit (70) zur Bestimmung der kontinuierlichen Erfassung so konfiguriert ist, dass sie bestimmt, dass der Detektor (15) das Erfassungsziel kontinuierlich erfasst, wenn der Detektor (15) das Erfassungsziel mit einer Rate erfasst, die größer oder gleich einem Schwellenwert innerhalb eines bestimmten Zeitbereichs ist, der länger als ein Erfassungsintervall des Detektors (15) ist.

13. Endoskopgerät nach einem der Ansprüche 1 bis 12, wobei der erste Beobachtungsmodus ein Normallicht-Beobachtungsmodus ist, bei dem Normallicht als erstes Beleuchtungslicht verwendet wird, und der zweite Beobachtungsmodus ein Speziallicht-Beobachtungsmodus ist, bei dem Speziallicht als zweites Beleuchtungslicht verwendet wird.

14. Endoskopgerät nach einem der Ansprüche 1 bis 12, wobei der erste Beobachtungsmodus ein erster Speziallicht-Beobachtungsmodus ist, bei dem ein erstes Speziallicht als erstes Beleuchtungslicht verwendet wird, und der zweite Beobachtungsmodus ein zweiter Speziallicht-Beobachtungsmodus ist, bei dem ein zweites Speziallicht, das sich von dem ersten Speziallicht unterscheidet, als zweites Beleuchtungslicht verwendet wird.

## Revendications

1. Appareil endoscopique comprenant :
une unité de source lumineuse (12) configurée pour émettre une première lumière d'illumination et une seconde lumière d'illumination correspondant respectivement à un premier mode d'observation et à un second mode d'observation ;
une unité d'imagerie (11) configurée pour capturer une image d'un sujet photographique irradié par la première lumière d'illumination ou la seconde lumière d'illumination ;
un détecteur (15) configuré pour détecter une cible de détection à partir d'images capturées séquentiellement par l'unité d'imagerie (11) ;
une unité de détermination de la détection continue (70) configurée pour déterminer si le détecteur (15) détecte continuellement la cible de détection ; et
une unité de commutation de mode d'observation (72) configurée pour passer du premier mode d'observation au second mode d'observation ;
une unité de calcul de la quantité de changement (73) configurée pour calculer une quantité de changement dans une région spécifique d'images capturées par l'unité d'imagerie (11) ; et
une unité de détermination de la quantité de changement (74) configurée pour déterminer si la quantité de changement calculée par l'unité de calcul de la quantité de changement (73) est comprise dans une valeur seuil,
dans lequel, dans un état où le premier mode d'observation est utilisé, l'unité de commutation du mode d'observation (72) est configurée pour passer automatiquement au second mode d'observation en réponse à l'unité de détermination de la détection continue (70) qui détermine que la cible de détection est détectée en continu et à l'unité de détermination de la quantité de changement (74) qui détermine que la quantité de changement est comprise dans la valeur seuil, la combinaison des deux indiquant que la section d'insertion de l'endoscope reste substantiellement stationnaire.

2. L'appareil endoscopique selon la revendication 1, dans lequel la région spécifique est une région entière d'une image capturée par l'unité d'imagerie (11).

3. L'appareil endoscopique selon la revendication 1, dans lequel la région spécifique est une région centrale d'une image capturée par l'unité d'imagerie (11).

4. L'appareil endoscopique selon la revendication 1, dans lequel la région spécifique est une région correspondant à la cible de détection, la région étant calculée sur la base des informations de détection provenant du détecteur (15).

5. L'appareil endoscopique selon l'une des revendications 1 à 4, dans lequel la quantité de changement calculée par l'unité de calcul de la quantité de changement (73) est une quantité de changement dans une position de la région spécifique.

6. L'appareil endoscopique selon l'une des revendications 1 à 4, dans lequel la quantité de changement calculée par l'unité de calcul de la quantité de changement (73) est une quantité de changement dans la taille de la région spécifique.

7. L'appareil endoscopique selon l'une des revendications 1 à 6, dans lequel, en réponse à l'écoulement d'une certaine période de temps après que l'unité de commutation de mode d'observation (72) est passée au second mode d'observation, l'unité de commutation de mode d'observation (72) est configurée pour passer au premier mode d'observation.

8. L'appareil endoscopique selon l'une des revendications 1 à 6, dans lequel, après que l'unité de commutation de mode d'observation (72) est passée au deuxième mode d'observation, l'unité de commutation de mode d'observation (72) est configurée pour passer au premier mode d'observation en réponse à l'unité de détermination de détection continue (70) qui détermine que la cible de détection n'est pas détectée de manière continue.

9. L'appareil endoscopique selon l'une des revendications 1 à 6, dans lequel, après que l'unité de commutation de mode d'observation (72) est passée au deuxième mode d'observation, l'unité de commutation de mode d'observation (72) est configurée pour passer au premier mode d'observation en réponse à l'unité de détermination de la quantité de changement (74) qui détermine que la quantité de changement est supérieure à la valeur seuil et qui détermine que la section d'insertion de l'endoscope reste substantiellement stationnaire.

10. L'appareil endoscopique selon l'une des revendications 1 à 6, dans lequel, après que l'unité de commutation de mode d'observation (72) est passée au second mode d'observation, l'unité de commutation de mode d'observation (72) est configurée pour passer au premier mode d'observation en réponse à la capture d'une image fixe.

11. L'appareil endoscopique selon l'une des revendications 1 à 10, dans lequel l'unité de détermination de la détection continue (70) est configurée pour déterminer que le détecteur (15) détecte continuellement la cible de détection en réponse à la détection consécutive par le détecteur (15) de la cible de détection dans une certaine plage de temps plus longue qu'un intervalle de détection du détecteur (15).

12. L'appareil endoscopique selon l'une des revendications 1 à 11, dans lequel l'unité de détermination de la détection continue (70) est configurée pour déterminer que le détecteur (15) détecte continuellement la cible de détection en réponse à la détection par le détecteur (15) de la cible de détection à un taux supérieur ou égal à une valeur seuil dans une certaine plage de temps plus longue qu'un intervalle de détection du détecteur (15).

13. L'appareil endoscopique selon l'une des revendications 1 à 12, dans lequel le premier mode d'observation est un mode d'observation à lumière normale dans lequel la lumière normale est utilisée comme première lumière d'éclairage, et le deuxième mode d'observation est un mode d'observation à lumière spéciale dans lequel la lumière spéciale est utilisée comme deuxième lumière d'éclairage.

14. L'appareil endoscopique selon l'une des revendications 1 à 12, dans lequel le premier mode d'observation est un premier mode d'observation à lumière spéciale dans lequel la première lumière spéciale est utilisée comme première lumière d'éclairage, et le deuxième mode d'observation est un deuxième mode d'observation à lumière spéciale dans lequel la deuxième lumière spéciale différente de la première lumière spéciale est utilisée comme deuxième lumière d'éclairage.
